# EUROPEAN PATENT SPECIFICATION

(11) **EP 2 671 084 B1**
(45) Date of publication and mention of the grant of the patent: **10.08.2016**
(21) Application number: 12703569.9
(22) Date of filing: 31.01.2012
(51) Int. Cl.: G01N 33/68

(54) **BIOMARKERS FOR OSTEOARTHRITIS**
BIOMARKER FÜR OSTEOARTHRITIS
BIOMARQUEURS DE L'ARTHROSE

(30) Priority: 31.01.2011 GB 201101639
(43) Date of publication of application: 11.12.2013
(60) Divisional application: 16174091.5
(73) Proprietor: L'Université de Liège, 4000 Liège (BE); Centre Hospitalier Universitaire Liègeois, 4000 Liège (BE); The University Of Bristol, Bristol BS8 1TH (GB)
(72) Inventor: SHARIF, Mohammed, Bristol BS2 8EJ (GB); MALAISE, Michel, 4000 Liège (BE); DE SENY, Dominique, 4000 Liège (BE)
(74) Representative: Henderson, Helen Lee
(86) International application number: PCT/GB2012/050203
(87) International publication number: WO 2012/104624

(56) References cited:
- WO-A1-2010/085606
- DATABASE BIOSIS [Online] BIOSCIENCES INFORMATION SERVICE, PHILADELPHIA, PA, US; June 2011 (2011-06), DE SENY DOMINIQUE ET AL: "Discovery and biochemical characterisation of four novel biomarkers for osteoarthritis", XP002676458, Database accession no. PREV201100321628 & ANNALS OF THE RHEUMATIC DISEASES, vol. 70, no. 6, June 2011 (2011-06), pages 1144-1152, ISSN: 0003-4967(print), DOI: DOI:10.1136/ARD.2010.135541

## Description

### Field of the Invention

The invention relates to biomarkers which may be used individually or in combination to diagnose osteoarthritis. The biomarkers are particularly useful as they may be used to diagnose the condition before the onset of significant symptoms.

### Background to the Invention

Osteoarthritis (OA) is one of the most common chronic joint diseases causing substantial health deficits¹ and it is becoming increasingly more prevalent as the population ages. Obesity is a major risk factor for developing OA and recent data suggest that there will be an epidemic of obesity-related osteoarthritis in the general population². Current diagnosis of OA depends on patient-reported pain and disability and is confirmed by plain radiography of affected joints. There are currently no biochemical tests available that can be used to diagnose, predict disease outcome or monitor treatment response. The availability of good biochemical markers would help with early monitoring of disease activity and outcome and will undoubtedly speed up development of OA-specific drugs.

OA is characterised by dysregulation of normal joint homeostasis that leads to intra-articular tissue degradation, attempted repair and inflammation mediated by cytokines and growth factors. The inventors and others have demonstrated that serum levels of macromolecules (biomarkers) can provide a way of measuring these processes³⁻⁸. The inventors' previous studies reporting extensive range of biomarkers have demonstrated that there are many macromolecules which are putative markers of radiographic disease outcome in knee OA while others have some diagnostic value⁹⁻¹¹. In their most recent study, the inventors looked at 20 different biomarkers and identified 8 that were associated with biological and pathological processes in knee OA. Several of these biomarkers also had diagnostic value and predicted radiographic disease progression, as well as pain and disability¹². However, these biomarkers (singly or in combination) lack specificity and sensitivity to discriminate individual patient from control or to suggest possible disease progression for patient over time. Therefore there is an urgent need to seek novel and more specific-biomarkers for investigation of OA and other joint diseases.

SELDI-TOF-MS (Surface Enhanced Laser Desorption/Ionisation-Time Of Flight-Mass Spectrometry) is a very powerful ProteinChip technology that differentially investigate levels of low molecular weight proteins (<20kDa) in biological fluids such as serum¹³⁻¹⁵. In studies of this type, unusually high or low proteins signals are of the main interest and considered as potential biomarkers of the disease process. SELDI ProteinChip surfaces include a set of classic chromatographic chemistry for capturing proteins according to their physico-chemical properties and displaying various protein profiles from the same studied biological sample, thus considerably increasing chances to catch specific biomarkers.

In the present study, serum samples from well to well characterised groups of OA patients, healthy controls and disease controls [(patients with rheumatoid arthritis (RA)] were analysed by SELDI-TOF-MS to search for novel and specific biomarkers for OA. The inventors were able to identify a number of markers that show particularly high specificity and selectivity for OA and which may be used individually or in combination with each other and/or other markers of OA.

### Summary of the Invention

There is provided a method of diagnosing osteoarthritis or monitoring the disease progression of osteoarthritis, comprising identifying the presence of an increased concentration of a marker selected from V65 vitronectin fragment or fragments, variants or degradation products thereof in a sample obtained from a subject, wherein the V65 vitronectin fragment or fragments, variants or degradation products thereof comprise a C-terminal end product of the V65 vitronectin submit in the heparin binding domain.

The term osteoarthritis is well known in the art. The term "diagnosing osteoarthritis" as used herein, is intended to mean confirming that an individual has OA, whether the individual is symptomatic or not. It encompasses identifying an individual who is likely to develop symptoms of OA, especially if treatment is not administered. The term "monitor disease progression" and associated terms mean to identify whether a disease has worsened or whether the subject's condition has improved. Such terms include monitoring a subject's response to treatment.

The subject is preferable a mammal, especially a primate. In one embodiment, the subject is a human.

The sample may be any sample obtainable from the subject, such as a blood, serum, urine or synovial fluid. It is preferably a serum sample or a sample of synovial fluid, especially from a joint thought to be, or likely to be affected by OA.

The markers used in the present invention inlcude C-terminal V65 vitronectin fragment or fragments or variants thereof, wherein the V65 vitronectin fragment or fragments, variants or degradation products thereof comprise a C-terminal end product of the V65 vitronectin submit in the heparin binding domain. They may be referred to collectively herein as "the markers" and a reference to "the markers" may be a reference to all of the markers.

The term C-terminal V65 vitronectin fragment means a C-terminal end product of the V65 vitronectin subunit in the heparin binding domain. The amino acid sequence of the C-terminal V65 vitronectin fragment is:

### SQRGHSRGRNQNSRRPS

C3f is a complement fragment and is well known in the art. It is a fragment released during the catabolic degradation of C3b by Factor H after C3 activation. Fragments or variants of C3f are modified, especially truncated, forms of C3f or forms in which one, two, three, four or more amino acids has been changed, deleted, added, substituted or otherwise modified. In particular fragments or variants include truncated fragments and variants in which 1, 2, 3, 4 or more amino acids have been removed from the C-terminal end of the peptide. Fragments and variants are preferably functional, that is to say they have similar properties to the entire protein and are bound specifically by similar binding molecules such as antibodies. The amino acid sequence of the C3f fragment is:

### SSKITHRIHWESASLLR

CTAPIII protein is well known in the art and also known as low affinity platelet factor-4. Fragments or variants of CTAPIII protein are modified forms of CTAPIII protein or forms in which one, two, three, four or more amino acids has been changed, deleted, added, substituted or otherwise modified. In particular fragments or variants include truncated fragments and variants in which 1, 2, 3, 4 or more amino acids have been removed from the N-terminal end of the peptide. Specific fragments include a fragment in which 4 amino acids have been removed from the N-terminal end of the peptide (β-thromboglobulin) and a fragment in which 2 amino acids have been removed from the N-terminal end of the peptide. The amino acid sequence of a CTAPIII protein is:

NLAKGKEESLDSDLYAELRCMCIKTTSGIHPKNIQSLEVIGKGTHCNQVEVIATLKD
GRKICLDPDA PRIKKIVQKK LAGDESAD

The concentration of the markers in the sample may be measured by any known means. It is preferably measured by SELDI-TOF-MS.

The term "increased concentration" preferably means that the concentration of the marker in question in the sample is higher than the expected concentration for that marker. The term "decreased concentration" preferably means that the concentration of the marker in question in the sample is lower than the expected concentration for that marker.

The expected concentration of a marker may be identified in a number of ways. For example, the sample may be compared with a comparable sample from another subject. If the concentration of a marker is higher or lower than that of the comparable sample, the concentration may be considered to be increased or decreased respectively.

The concentration of a marker in the sample may be compared with a standard concentration of the marker or range of marker concentrations appropriate for the sample type and the subject from whom the sample is collected. Reference ranges may be established by testing a large number of comparable samples from a number of subjects over a range of ages. Such reference ranges may allow the determination of the distribution of marker concentrations. Comparable samples are the same type of sample as each other. For example, comparable samples are taken from subjects of the same species, gender and age group The samples should also be taken at the same time of day, as diurnal variations may affect marker concentrations. Equally, synovial fluid samples should be compared with other synovial fluid samples, serum samples with other serum samples etc.

The increase or decrease in concentration of each marker is preferably statistically significant. For example, the concentration change is at least 5%, preferably at least 10%, more preferably at least 15%, more preferably at least 20%, more preferably at least 25%, more preferably at least 30%, more preferably at least 35%.

Alternatively, the sample may be compared with a projected marker concentration, established by testing the same subject at an earlier date and predicting the marker concentration that is likely to be observed if the subject has OA. The prediction may simply be an increase or decrease in marker concentration, or it may be possible to quantify the likely change.

The method may involve testing for V65 vitronectin fragment and C3f and/or CTAPIII. The method may also include testing for other markers of OA or related conditions, such as TNF-α, MMP-3 COMp and CTX2.

The invention will now be described in detail, by way of example only, with reference to the figures.

### Figures

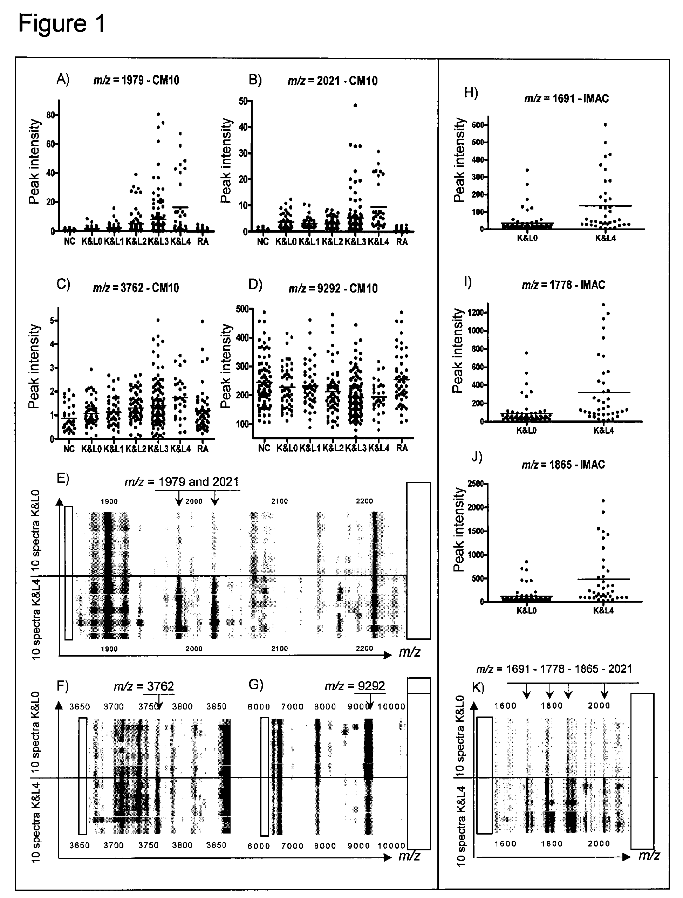

Figure 1: A),B),C),D) - Peak intensity distribution of biomarkers detected after Validation 1 study on CM10 (m/z = 1979, 2021, 3762 and 9292) through the 7 groups of patients (NC, K&L0 1, 2, 3, 4 and RA). E), F) and G) - Gel view spectra provided by 20 patients with OA (10 with K&L0 and 10 with K&L4) representing 1979 and 2021, 3762 and 9292 m/z markers respectively. H),I) and J) - Peak intensity distribution of biomarkers detected after Validation 2 study on IMAC-Ni (m/z = 1691, 1778 and 1865) through K&L0 and K&L4 groups. K) - Gel view spectra provided by 20 patients with OA (10 with K&L0 and 10 with K&L4) representing 1691, 1778, 1865 and 2021 m/z markers.

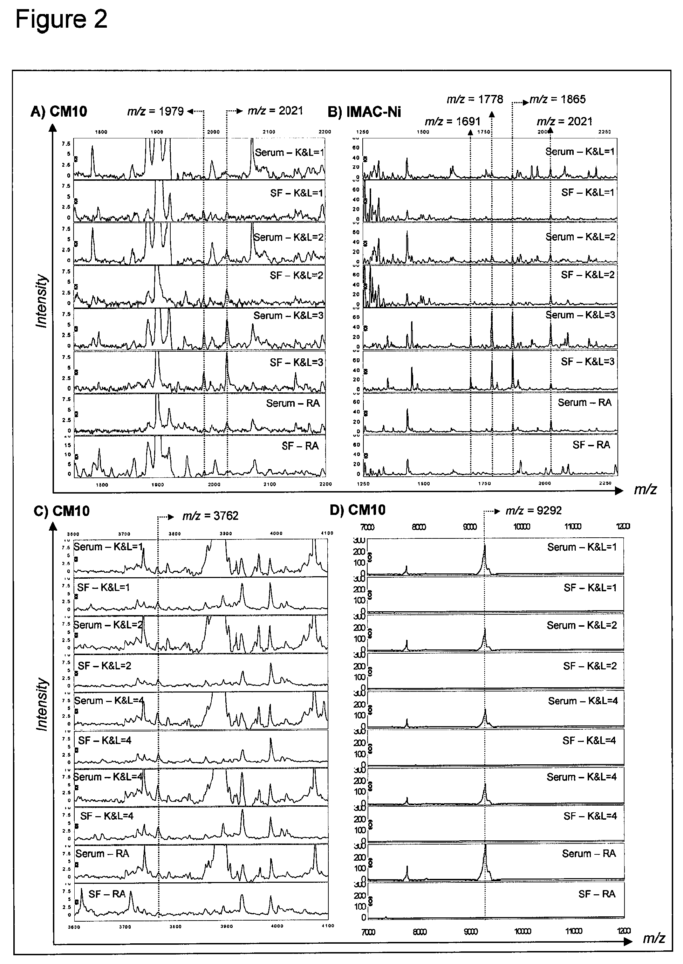

Figure 2: Proteomic study on paired serum and synovial fluid spectra provided from OA (K&L1 to K&L4) and RA patients, illustrating presence or absence of peaks at m/z values of 1979 (A), 2021 (A and B), 1691 (B), 1778 (B), 1865 (B), 3762 (C) and 9292 (D) in both fluids.

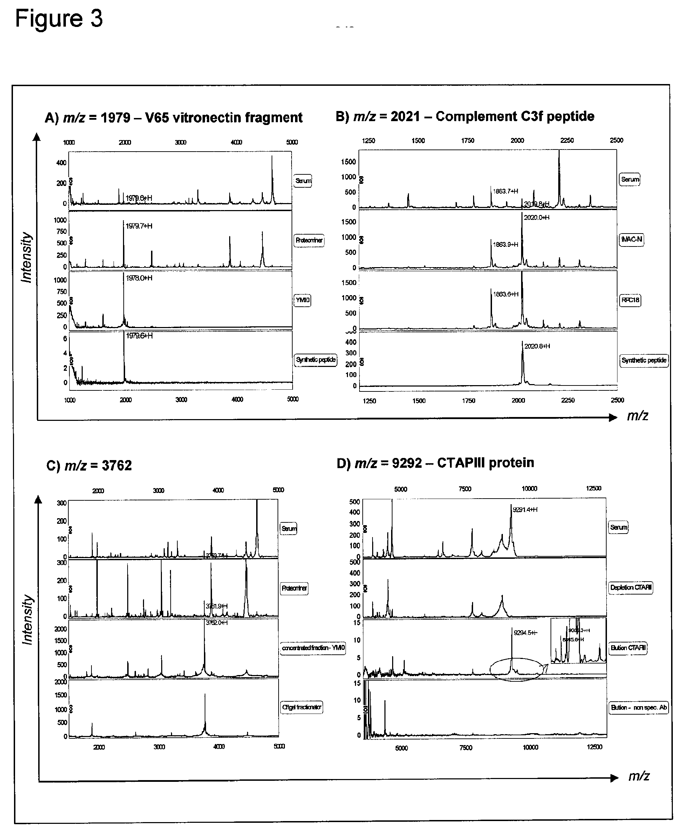

Figure 3: Purification and identification of the protein fragments at m/z value of 1979, 2021, 3762 and 9292 as V65 vitronectin fragment, complement fragment C3f, remains unknown (m/z = 3762) and CTAPIII proteins. A) Enrichment of the V65 vitronectin fragment with the Proteominer kit and the ultrafiltration process on YM-10, and identification by the use of proteinchip and the MALDI-TOF-MSMS. Synthetic peptide was loaded on the last spectra. B) Identification of complement C3f by µHPLC-ESI-TRAP-MSMS after purification on IMAC-Ni column and C18 resin. Synthetic peptide was also loaded on the last spectra. C) Enrichment of the peptide corresponding to the 3762 m/z value using the Proteominer kit and concentration process on YM-10 and D) Identification of CTAPIII proteins by depletion of serum using specific and non specific antibodies followed by elution from the immune complexes.

### Patients and Methods

### Patients

284 patients with knee OA were enrolled through community questionnaires, primary care consultations, hospital outpatient clinics^{9,10} and classified according to their K&L score (16). 25 RA patients fulfilling the 1987 American College of Rheumatology criteria (17) and 36 healthy individuals [(referred as negative controls (NC)] were included into the study as controls. OA and NC serum samples were distributed in two sets of samples: the analysis set (130 OA and 20 NC) and the validation set (154 OA and 16 NC). RA samples (n = 25) were only included in the validation set. 20 paired serum/synovial fluids samples (12 OA and 7 RA patients) were used for correlation between both fluids. Demographic and epidemiologic data of these patients are summarized in Table 1. The study protocol was approved by the institutional review boards (Research Ethics Committee) of both the United Bristol Healthcare NHS trust, Bristol, UK, and the University Hospital, CHU Liege, Belgium.

### Serum samples and synovial fluids

Serum samples were collected in plain glass tubes, allowed to coagulate, and centrifuged at 2,800 revolutions per minute for 10 minutes. Synovial fluids were processed using the same experimental conditions as serum. Supernatants were aliquoted and immediately frozen at -80°C until required for SELDI-TOF-MS analysis.

### ProteinChip array preparation

The 150 serum samples used for analysis (Table 1a) were loaded in duplicates on two kinds of ProteinChip arrays (BioRad, USA) in order to capture a wide range of potential biomarkers: an anionic (CM10) and an immobilized metal affinity capture covalently bound with Ni²⁺ (IMAC-Ni). One year later, a first validation study (Validation 1) was performed in the same experimental and time frame conditions, with an independent set of 195 serum samples (Table 1b) and loaded on CM10 arrays only. A second validation study (Validation 2) was performed the following month using a subset of 39 serum samples from Validation 1 including K&L0 and K&L4 stages only (Table 1b). Validation 2 was performed in a day on both CM10 and IMAC-Ni arrays. Finally, paired serum and synovial fluid samples from 12 OA (K&L1 to 4) and 7 RA patients (Table 1c) were loaded on CM10 arrays and analysed to check for the new biomarkers' presence in both fluids.

For all studies, serum samples and synovial fluids were first denatured within 1.5 volume of 7M urea, 2M thiourea, 2% CHAPS and 50mM Tris (pH9), and then diluted 6 times in the following buffers: 100mM Tris, pH9 (CM10) and 100mM phosphate buffer containing 250mM NaCl, pH7 (IMAC-Ni). ProteinChip arrays were prepared according to manufacturer's instructions and as described previously^{18,19} 1µL of α-cyano-4-hydroxycinnamic acid (CHCA, Biorad) diluted 2 times and 1µL of sinapinic acid (SPA, BioRad) were added to each spot of CM10 and IMAC-Ni, respectively. For the validation 2 performed on IMAC-Ni, CHCA was used to better visualize small peptides. Synovial fluids were loaded as serum samples. The arrays were analysed with a PBSII and a PCS4000 ProteinChip reader for the analysis and the validation study, respectively. Spectra were acquired on an m/z (mass to charge ratio) of 1800-20000 and 1500-20000 for CM10 and IMAC-Ni, respectively.

ProteinChips arrays were read over the course of a week to limit variability across the time. Standardization of experimental conditions was carried out in an effort to minimize effects of irrelevant sources of fluctuation. Serum samples were applied randomly in order to avoid the detection of artefacts due to experimental handling

### Protein purification for biomarkers identification

The proteomic study detected 4 proteins at different m/z ratios as potential novel biomarkers and a range of biochemical methods were used to characterize and identify these markers. The fragment at 1979 m/z value was first enriched from 1mL of serum with the Proteominer^{®} kit (BioRad) according to manufacturer's instructions, then dialysed against a phosphate buffered saline (PBS) solution (Lonza, Belgium) and filtered using YM10 filters (Microcon, Millipore, MA). Peptides in the flow-through fraction were applied on CM10 arrays according to the previously described experimental conditions. The ProteinChip array was then analysed by MALDI-TOF mass spectrometry (Ultraflex II, Bruker) for identification of this peptide. The second peptide at 2021 m/z value was purified using 100µL of serum on an IMAC-Ni column chromatography (Affiland, Belgium) with phosphate buffer 100mM, imidazole 100mM, NaCl 0.3M, pH7.5 as elution solution. The eluted peptides were concentrated on dynabeads RPC18 (Invitrogen, Belgium) and eluted with 20µL of 50% acetonitrile to be identified by µHPLC-ESI-TRAP-MSMS (Esquire, Bruker). The peptide at 3762 m/z value was also enriched using the proteominer kit (BioRad) and concentrated on YM10 filters (Microcon). The concentrated solution was loaded on the Offgel fractionator (Agilent) according to manufacturer's instructions. Collected fractions were analysed on H50 ProteinChip array (BioRad). The fourth peptide at 9292 m/z value was identified by immunodepletion using 50µL of serum. Serum was immunodepleted using Protein G-Agarose beads (Santa Cruz) coated with an anti-CTAPIII (anti-connective tissue-activating peptide III) (Abcam, UK) monoclonal antibody. The protein fragment was eluted using 100mM acetic acid in 30% ACN, and detected on IMAC-Ni ProteinChip array (BioRad). The anti-cystatin B monoclonal antibody (Santa Cruz) was used as negative control.

### Peptides synthesis

Synthetic peptides of complement C3f and V65 vitronectin fragment, were synthesized by solid-phase peptide synthesis (Eurogentec, Belgium) and applied on ProteinChip arrays to be compared to their corresponding purified peptides.

### Correlation with currently available biomarker assays

In order to further characterise the 4 novel biomarkers, we correlated biomarkers peak intensities at 1979, 2021, 3762 and 9292 m/z values with 20 different biochemical markers associated with cartilage, bone, and synovial tissue metabolism. These markers had been previously measured by ELISA based assays (12) in 93 of the 154 OA serum samples used in the validation 1 set, and were equally distributed among the K&L scores.

### Statistical analyses

Pre-processing of spectra involving calibration, baseline subtraction and normalisation were completed before statistical analysis. Peak detection was performed using ProteinChip Biomarker Wizard software 3.0 (BioRad). Data were analysed by two statistical approaches: a nonparametric Mann-Whitney U test and a machine-learning algorithm called *Extra-Trees N.* The latter approach is a decision-tree multivariate analysis used to build classification models and to rank peaks according to their relative contribution in the classification of 2 groups (20). In this model, m/z peak values were ranked in relation to their percentage of importance [Imp(%)] for differentiating K&L0 from K&L4 groups. First values represent potential biomarkers involved in the development of OA. *P* values are associated to this ranking and are considered significant if ≤ 0.05. For determining the association with currently available biomarkers, ranked data were used to calculate Spearman correlation coefficients. *P* values < 0.05 were considered statistically significant.

### Results

### Reproducibility.

Peaks intensity variability on SELDI protein profiles provided by the same serum sample applied 8 times on the same chip (intra-experiment) or applied consecutively during 7 days (inter-experiment) were determined by coefficient of variations (CVs) as described by de Seny et *al.* (19). Intra-chip variations were evaluated on CM10 arrays at 12.4% and 10% for analysis and validation study, respectively and on IMAC-Ni arrays at 13.3% for analysis study. Inter-chip variations after 7 days were determined on CM10 arrays at 18.5% and 30% for analysis and validation study, respectively and on IMAC-Ni arrays at 24.1% for analysis study. The variability of biomarkers at m/z peak value of 1979, 2021, 3762 and 9292 was also determined during the validation study using a K&L0 and K&L4 quality control serum sample. CVs were found to be in the range of 5.1% to 10.4% for intra-experiment and from 27.5% to 33.6% for inter-experiment. Calculated CVs are in the range of published data.

### Serum analysis study

From the 130 OA and 20 NC serum samples (Analysis set, Table 1a), 300 spectra were obtained on both CM10 and IMAC-Ni ProteinChip arrays. Biomarker Wizard software resolved 156 and 134 peaks on CM10 and IMAC-Ni, respectively. Ranking of biomarkers in the top 19 is summarized in Table 2 of the Analysis section and is made according to the Imp (%) given by the multivariate analysis. A *P*-value for each biomarker was also calculated.

### Serum validation studies

A new proteomic study (Validation 1) was designed with an independent set of samples to confirm the robustness of biomarkers detected after analysis study. Validation 1 was performed on CM10 ProteinChip arrays only. This study used 154 OA serum samples (Table 1b) classified according to their K&L score, plus 16 sera from healthy individuals (NC group) and 25 sera from RA patients. All samples were loaded in duplicate on CM10, generating a total of 390 spectra. Same statistical approaches as employed for the analysis set were used for peaks ranking [Table 2 - Validation 1 section].

Analysis and validation studies comparison allowed us to bring out 4 potentially novel biomarkers at 1979, 2021, 3762 and 9292 m/z values (Figure 1). Peak intensities distribution in NC, in OA (K&L0 to 4) and in RA groups are shown in Figure 1A, B, C, D.

Peak intensities at 1979 and 2021 m/z values were found significantly (P<0.0001) increased in all OA samples compared to NC and RA samples and further increased with increasing K&L scores (Figure 1A,B). With a peak intensity's cut-off of 2.4 (mean + 2 SD of controls), sensitivity for the 1979 m/z peak was of 29% in K&L0, 41% in K&L1, 38% in K&L2, 57% in K&L3 and 66% in K&L4, while its specificity was of 97%. With a peak intensity's cut-off of 1.5 (mean + 2 SD of controls), sensitivity for the 2021 m/z peak was of 91% in K&L0, 100% in K&L1, 72% in K&L2, 87% in K&L3 and 93% in K&L4, while its specificity was of 92%. Intensities at 3762 and 9292 m/z values were significantly higher (m/z=3762) (P=0.014) or lower (m/z=9292) (P=0.004) in OA patients than in control groups, and the former (Figure 1C) increased with increasing K&L scores of OA patients while the latter (Figure 1d) decreased with increasing K&L scores. Figure 1E, F and G shows the four biomarkers in gel view spectra from 10 OA patients with K&L0 and 10 with K&L4. The figure shows that the biomarkers with m/z values at 1979, 2021 and 3762 are barely detectable in K&L0 but they are present at much higher concentrations in serum samples from patients with K&L scores of 4, while the opposite is true for the biomarker at m/z value of 9292.

Validation 2 was performed in a single day on the 39 K&L0 and K&L4 patients used in Validation 1, to ensure that these 4 biomarkers were not artefacts due to the time spent during Validation 1 study performed over 7 days. It allowed us to discard any technical ambiguities and to strengthen the validity of our 4 biomarkers still showing *P* values below 0.05 [Table 2 - Validation 2]. Moreover, due to the use of CHCA that better visualize small peptides, new biomarkers were detected at 1691, 1778 and 1865 m/z values on IMAC-Ni. These new biomarkers are illustrated in Figure 1H,I,J,K and were found to be variants of the 2021 m/z peak as further discussed below. The 2021 m/z biomarker can also be visualized in gel view K&L4 spectra (IMAC-Ni arrays) (Figure 1K).

### Synovial fluid analysis study

Peaks at 1979, 2021 and 3762 m/z values were simultaneously identified in both serum and synovial fluids samples of OA patients at different K&L scores (Figure 2). Peaks at 9292 m/z value were only detected in serum but not in synovial fluids of OA and RA patients. The 1979 m/z peak was also present in 2/7 spectra from RA synovial fluid but not in its corresponding serum sample (data not shown). Finally, peaks corresponding to variants of the 2021 m/z biomarker were also detected in both serum and synovial fluids samples of all OA patients, but also in 3/7 RA patients.

### Biomarkers identification

Proteins at 1979 and 3762 m/z values were purified (Figure 3A and C) and applied on ProteinChip arrays (CM10 and H50, respectively) to be analysed by MALDI-TOF-TOF mass spectrometry. The 1979 m/z value was identified as a C-terminal end product of the V65 vitronectin subunit when the 3762 m/z value remained undetectable due to its too high hydrophobicity. The 2021 m/z protein was identified after purification procedures (Figure 3B) by µHPLC-ESI-TRAP-MSMS as complement C3f peptide. Protein identification at 9292 m/z value was carry out by immunodepletion using protein G-agarose beads coated with anti-CTAPIII antibodies (Figure 3D). CTAPIII was eluted from its immune complex and applied on ProteinChip arrays to be analysed by SELDI-TOF-MS.

Peaks at 1691, 1778, 1865 and 2021 m/z values were found clustered on spectra provided by IMAC-Ni arrays and therefore are variants (truncated form at the C-terminal end by 3, 2, and 1 amino acid, respectively) of complement C3f peptides.

Same observation was made for 8862, 9060 and 9292 m/z peaks as these proteins were eluted simultaneously from their immune complex. 8862 m/z value is a 4 amino acids truncated form at the N-terminal end of CTAPIII and is better known as β-thromboglobulin (β-TG) protein (peptide mass of 8865 Da). 9060 m/z peak is a 2 amino acids truncated form at N-terminal end of CTAPIII (peptide mass of 9064). Furthermore, peaks at m/z values of 4430, 4530 and 4644 represent doubly charged (2H+) forms of 8862, 9060 and 9292 m/z values. These forms were found statistically significant in the analysis study but not in the validation one.

### Correlation with currently available biomarker assays

Peak intensities corresponding to 1979 (V65 vitronectin fragment), 2021 (C3f), 3762 (unknown) and 9292 (CTAPIII) m/z values and detected in spectra generated by 93 OA serum samples, were correlated to a wide range of currently available biomarkers (Table 3). V65 vitronectin fragment was positively correlated with C-telopeptide of type II collagen (CTXII) and negatively with aggrecan and pro-matrix metalloprotein 1 (pro-MMP1). C3f peptide was negatively correlated with aggrecan and pyridinoline (pyd). The protein fragment at 3762 m/z value showed positive correlation with cartilage glycoprotein 39 (YKL-40), MMP-3, pyd and deoxypyridinoline (dpd), and negatively correlated with keratan sulphate epitope 5D4 (KS). Finally, CTAPIII was positively correlated with MMP-3, tumor necrosis factor α (TNF-α) and aggrecan.

### Discussion

SELDI-TOF-MS technology is a very powerful technique to investigate proteome of hundreds serum samples or synovial fluids and to detect potential protein biomarkers involved in OA pathology. Using this proteomic approach, we have detected 4 novel biomarkers for OA, and all of them appeared to discriminate OA patients from controls (NC and RA). Two of them have been identified as the C-terminal end product of V65 vitronectin subunit and complement C3f peptide. A third one at 3762 m/z value remains unidentified due to its too high hydrophobicity. These three biomarkers were found up-regulated in sera of patients with severe OA and were also detected in synovial fluids from the same patients. The fourth novel biomarker was identified as a platelet-specific protein, CTAPIII, and was down-regulated in serum of severe OA patients.

### V65 vitronectin fragment

The 1979 m/z peak identified as a C-terminal end product of the V65 vitronectin subunit in the heparin binding domain, was predominantly detected in CM10 spectra of OA patients with worst disease (K&L3 and 4) in both analysis and validation studies. However, peak intensities were significantly higher in all OA subsets, including K&L0, compared to controls (NC and RA) and further increased with increasing K&L scores. Preliminary proteomics study on paired serum samples and synovial fluids of OA patients revealed the presence of this fragment in both fluids. The 1979 m/z peak was also detected in 2/7 spectra from RA synovial fluid but not in its corresponding serum sample (data not shown). Accordingly, serum V65 vitronectin fragment may be derived from joint fluid/tissues and appeared to be an interesting marker of OA with a global sensitivity (all OA subsets) of 47% and a specificity of 97%.

Vitronectin is a cell adhesion and spreading factor found in serum and in many tissues including cartilage and synovium²¹. It is recognized by αVβ3 integrin receptor. Interaction between vitronectin through its arginine glycine and aspartic acid motif (RGD)²² has been well described²³⁻²⁵. Highly expressed αVβ3 integrins have been detected on bone-resorbing osteoclasts and were shown to play an essential role in mediating osteoclasts attachment to bone matrix allowing active bone resorption^{23,26,27}. A weak expression of αVβ3 integrin receptor has also been observed on chondrocytes²⁸ and it has been described as a major player in the regulation of inflammatory mediators (1L-1β, NO and PGE₂) in osteoarthritis-affected cartilage²⁹ but it is not thought to be involved in the adhesion of chondrocytes to cartilage³⁰ or transmission for chondrocytes dedifferentiation³¹. The αVβ3 integrin is also found in other joint tissues such as fibroblast-like synoviocytes³².

Since cartilage loss and mild to moderate synovial inflammation are recognised features of OA especially at advanced stages (K&L3 and 4) of the disease, V65 vitronectin fragment could play an important role in the pathogenesis of OA. Moreover, in the present study, OA progression is characterized by elevated serum levels of V65 vitronectin fragments and CTX-II, and low serum levels of aggrecan and pro-MMP-1, a typical pattern of progressive OA when the cell number declines³³, therefore V65 vitronectin fragment may also be an important marker of disease outcome in OA.

### Complement C3f peptide and variants

The 2021 m/z peak identified as complement C3f peptide was predominantly detected in IMAC-Ni²⁺ spectra from OA patients with worst disease (K&L3 and 4) in both analysis and validation studies. Moreover, C3f peptide serum levels were significantly higher in all OA subsets, including K&L0, compared to controls (NC and RA) and further increased with increasing K&L scores. A second validation study (Validation 2) was performed in a single day to circumvent false positives biomarkers detection due to extensive sample handling procedures as described by West-Norager et *al.*³⁵, and was considered as a quality control study. CHCA instead of SPA matrix was, however, used in Validation 2 to better visualize small peptides below 2000 Da. Three peaks, at 1691, 1778 and 1865 m/z values were therefore detected with a high discriminatory power when comparing OA patients with worst radiographic OA (K&L score 4) to patients with no radiographic OA (K&L score 0). These peaks were clustered on spectra and their m/z values correspond to truncated variants of complement C3f. C3f peptide is a fragment released during the catabolic degradation of C3b by Factor H after C3 activation³⁶. C3f is further degraded to form C3f-des-Arg (peptide mass of 1865 Da) and variants by carboxypeptidase N. Preliminary proteomics study on serum samples and synovial fluids of OA patients revealed the presence of C3f peptides in both fluids, but also in 3/7 paired RA serum/synovial fluid (data not shown). However, as for V65 vitronectin fragment, C3f peptide was found to have some diagnostic values in OA, with a global sensitivity (all OA subsets) of 87% and a specificity of 92%. Furthermore, C3f peptide serum levels were significantly (and positively) correlated with V65 vitronectin serum levels in OA patients tested. Interestingly, vitronectin is also involved in the complement cascade, inhibiting the assembly of the membrane attack complex. Finally, C3f biomarker showed also significant negative correlations with serum levels of aggrecan and pyd.

Significant pathophysiological roles could be played by complement C3f in cartilage and other joint tissue metabolism. Indeed, complement components can be found in healthy cartilage as they are expressed by chondrocytes. However, their production may be increased in the presence of pathological mediators. Cartilage mainly consists of ECM made with the highly negatively charged proteoglycan (aggrecan). ECM is found settled in networks of collagen fibers, which contain many other bound molecules such as small leucine-rich repeat proteins (SLRPs). Some of these SLRPs, such as fibromodulin (FMOD) and osteoadherin (OSAD), interact with the globular head domain of C1q to further activate the classical and alternative pathways of complement factors^{37,38}. FMOD and OSAD, as well as chondroadherin (CHAD) also interact with Factor H. In OA pathology, where the integrity of the ECM is compromised by proteolytic degradation, SLRPs are released into synovial fluid becoming exposed to complement factors promoting its activation and, hence, further opsonisation of self-tissues, anaphylaxis and general inflammation. In more severe cases (K&L grades 3 and 4) serum C3f fragments were higher while the levels of circulating aggrecan and pyd were lower. This observation is consistent with sharp decline of proteoglycan synthesis and the promotion of bone remodelling normally seen in OA patients with progressive disease. Our further studies will aim to determine whether there is a direct correlation between these SLRPs and C3f peptides. Complement C3f peptides had been detected by another recent proteomics study³⁹ using purification of short peptides with C 18-bound magnetic beads, in sera of patients with systemic sclerosis (SSc). The authors described a predominance of the C3f-des-Arg form in sera of SSc patients.

### m/z = 3762

Peak intensities at 3762 m/z value were significantly increased in OA patients as compared to NC and/or RA patients, and also increased with increasing K&L scores. We have not been able to identify this protein of interest because of its high hydrophobicity and difficulty to be ionized. However, it was significantly and positively correlated with serum YKL-40, MMP-3, pyd and dpd levels, and negatively with KS. Therefore, measurement of this biomarker in serum may reflect overall changes in major joint tissues since it has been associated with cartilage damage (YKL-40, KS), synovial inflammation (MMP-3) and bone remodelling (pyd, dpd) (40-42).

### CTAPIII and variants

CTAPIII peak intensities (9292 m/z value) were significantly lower in the OA patients population compared to controls. And among OA subsets, CTAPIII remained significantly reduced in OA patients with the worst radiographic diseases (K&L score 3 or 4). Variants [β-TG (8862 m/z value), truncated form at 9060 m/z value, doubly charged forms (2H+) at 4644, 4430 and 4530 m/z value] were also detected as discriminatory biomarkers in the analysis study. However, CTAPIII was the only biomarker having a statistically discriminant P value in both analysis and validation studies. CTAPIII and variants were only detected in serum samples but not in synovial fluids suggesting that these peptides are not locally produced in joints and cannot be therefore joint tissue specific. On the other hand, CTAPIII and variants could be markers of systemic inflammation, a notion that would be supported by positive correlations with serum levels of TNF-α and MMP-3. In other words, severe stages of knee OA are characterized by low serum levels of CTAPIII, TNF-α and MMP-3, also a typical pattern of progressive OA, when cells number declines.

### Conclusion

Our proteomic study has detected 4 novel OA biomarkers. At least two of them, V65 vitronectin fragment with a specificity of 97% and complement C3f with a specificity of 92%, can discriminate OA patients from controls (NC and RA) and are likely potential serum diagnostic markers for OA. The remaining unidentified protein at m/z 3762 was up-regulated while the CTAPIII protein was down-regulated in the more severe stages of osteoarthritis. All 4 novel serum biomarkers have significant correlations with clusters of parameters reflecting the decline in cartilage metabolism, in local inflammation and the continuous bone remodelling as osteoarthritis progresses. These correlations suggest that these new serum biomarkers might also have pathophysiological relevance and could represent interesting new starting points to better understand OA.

### References

1. Guccione AA, Felson DT, Anderson JJ, et al. The effects of specific medical conditions on the functional limitations of elders in the Framingham Study. Am J Public Health 1994;84:351-8.
2. Allman-Farinelli MA, Aitken RJ, King LA, Bauman AE. Osteoarthritis--the forgotten obesity-related epidemic with worse to come. Med J Aust 2008;188:317.
3. Garnero P, Ayral X, Rousseau JC, et al. Uncoupling of type II collagen synthesis and degradation predicts progression of joint damage in patients with knee osteoarthritis. Arthritis Rheum 2002;46:2613-24.
4. Petersson IF, Boegard T, Dahlstrom J, Svensson B, Heinegard D, Saxne T. Bone scan and serum markers of bone and cartilage in patients with knee pain and osteoarthritis. Osteoarthritis Cartilage 1998;6:33-9.
5. Petersson IF, Boegard T, Svensson B, Heinegard D, Saxne T. Changes in cartilage and bone metabolism identified by serum markers in early osteoarthritis of the knee joint. Br J Rheumatol 1998;37:46-50.
6. Poole AR, Ionescu M, Swan A, Dieppe PA. Changes in cartilage metabolism in arthritis are reflected by altered serum and synovial fluid levels of the cartilage proteoglycan aggrecan. Implications for pathogenesis. J Clin Invest 1994;94:25-33.
7. Saxne T, Heinegard D. Cartilage oligomeric matrix protein: a novel marker of cartilage turnover detectable in synovial fluid and blood. Br J Rheumatol 1992;31:583-91.
8. Sharif M, Saxne T, Shepstone L, et al. Relationship between serum cartilage oligomeric matrix protein levels and disease progression in osteoarthritis of the knee joint. Br J Rheumatol 1995;34:306-10.
9. Sharif M, George E, Shepstone L, et al. Serum hyaluronic acid level as a predictor of disease progression in osteoarthritis of the knee. Arthritis Rheum 1995;38:760-7.
10. Sharif M, Granell R, Johansen J, Clarke S, Elson C, Kirwan JR. Serum cartilage oligomeric matrix protein and other biomarker profiles in tibiofemoral and patellofemoral osteoarthritis of the knee. Rheumatology (Oxford) 2006;45:522-6.
11. Sharif M, Kirwan JR, Elson CJ, Granell R, Clarke S. Suggestion of nonlinear or phasic progression of knee osteoarthritis based on measurements of serum cartilage oligomeric matrix protein levels over five years. Arthritis Rheum 2004;50:2479-88.
12. Davis CR, Karl J, Granell R, et al. Can biochemical markers serve as surrogates for imaging in knee osteoarthritis? Arthritis Rheum 2007;56:4038-47.
13. De Bock M, de Seny D, Meuwis MA, et al. Challenges for biomarker discovery in body fluids using SELDI-TOF-MS. J Biomed Biotechnol;2010:906082.
14. Tang N, Tornatore P, Weinberger SR. Current developments in SELDI affinity technology. Mass Spectrom Rev 2004;23:34-44.
15. Weinberger SR, Boschetti E, Santambien P, Brenac V. Surface-enhanced laser desorption-ionization retentate chromatography mass spectrometry (SELDI-RC-MS):
   a new method for rapid development of process chromatography conditions. J Chromatogr B Analyt Technol Biomed Life Sci 2002;782:307-16.
16. Kellgren JH, Lawrence JS. Radiological assessment of osteo-arthrosis. Ann Rheum Dis 1957;16:494-502.
17. Arnett FC, Edworthy SM, Bloch DA, et al. The American Rheumatism Association 1987 revised criteria for the classification of rheumatoid arthritis. Arthritis Rheum 1988;31:315-24.
18. de Seny D, Fillet M, Meuwis MA, et al. Discovery of new rheumatoid arthritis biomarkers using the surface-enhanced laser desorption/ionization time-of-flight mass spectrometry ProteinChip approach. Arthritis Rheum 2005;52:3801-12.
19. de Seny D, Fillet M, Ribbens C, et al. Monomeric calgranulins measured by SELDI-TOF mass spectrometry and calprotectin measured by ELISA as biomarkers in arthritis. Clin Chem 2008;54:1066-75.
20. Geurts P, Fillet M, de Seny D, et al. Proteomic mass spectra classification using decision tree based ensemble methods. Bioinformatics 2005;21:3138-45.
21. Rosenblum G, Carsons S. Quantitation and distribution of vitronectin in synovial fluid and tissue of patients with rheumatic disease. Clin Exp Rheumatol 1996;14:31-6.
22. Xiong JP, Stehle T, Diefenbach B, et al. Crystal structure of the extracellular segment of integrin alpha Vbeta3. Science 2001;294:339-45.
23. Horton MA. The alpha v beta 3 integrin "vitronectin receptor". Int J Biochem Cell Biol 1997;29:721-5.
24. Nakamura I, Duong le T, Rodan SB, Rodan GA. Involvement of alpha(v)beta3 integrins in osteoclast function. J Bone Miner Metab 2007;25:337-44.
25. Rinaldi N, Weis D, Brado B, et al. Differential expression and functional behaviour of the alpha v and beta 3 integrin subunits in cytokine stimulated fibroblast-like cells derived from synovial tissue of rheumatoid arthritis and osteoarthritis in vitro. Ann Rheum Dis 1997;56:729-36.
26. Nesbitt S, Nesbit A, Helfrich M, Horton M. Biochemical characterization of human osteoclast integrins. Osteoclasts express alpha v beta 3, alpha 2 beta 1, and alpha v beta 1 integrins. J Biol Chem 1993;268:16737-45.
27. Shinar DM, Schmidt A, Halperin D, Rodan GA, Weinreb M. Expression of alpha v and beta 3 integrin subunits in rat osteoclasts in situ. J Bone Miner Res 1993;8:403-14.
28. Loeser RF. Integrin-mediated attachment of articular chondrocytes to extracellular matrix proteins. Arthritis Rheum 1993;36:1103-10.
29. Attur MG, Dave MN, Clancy RM, Patel IR, Abramson SB, Amin AR. Functional genomic analysis in arthritis-affected cartilage: yin-yang regulation of inflammatory mediators by alpha 5 beta 1 and alpha V beta 3 integrins. J Immunol 2000; 164:2684-91.
30. Kurtis MS, Schmidt TA, Bugbee WD, Loeser RF, Sah RL. Integrin-mediated adhesion of human articular chondrocytes to cartilage. Arthritis Rheum 2003;48:110-8.
31. Goessler UR, Bugert P, Bieback K, et al. In vitro analysis of differential expression of collagens, integrins, and growth factors in cultured human chondrocytes. Otolaryngol Head Neck Surg 2006;134:510-5.
32. Nam EJ, Sa KH, You DW, et al. Up-regulated transforming growth factor beta-inducible gene h3 in rheumatoid arthritis mediates adhesion and migration of synoviocytes through alpha v beta3 integrin: Regulation by cytokines. Arthritis Rheum 2006;54:2734-44.
33. Di Cesare PE. Pathogenesis of osteoarthritis. In: Firestein GS, Budd RC, Harris Jr. ED, McInnes IB, Ruddy S, Sergent JS, eds. Kelley's textbook of rheumatology. Philadelphia, 2008:1525-46.
34. Imai K, Shikata H, Okada Y. Degradation of vitronectin by matrix metalloproteinases-1, -2, -3, -7 and -9. FEBS Lett 1995;369:249-51.
35. West-Norager M, Kelstrup CD, Schou C, Hogdall EV, Hogdall CK, Heegaard NH. Unravelling in vitro variables of major importance for the outcome of mass spectrometry-based serum proteomics. J Chromatogr B Analyt Technol Biomed Life Sci 2007;847:30-7.
36. Ganu VS, Muller-Eberhard HJ, Hugli TE. Factor C3f is a spasmogenic fragment released from C3b by factors I and H: the heptadeca-peptide C3f was synthesized and characterized. Mol Immunol 1989;26:939-48.
37. Sjoberg A, Onnerfjord P, Morgelin M, Heinegard D, Blom AM. The extracellular matrix and inflammation: fibromodulin activates the classical pathway of complement by directly binding C1q. J Biol Chem 2005;280:32301-8.
38. Sjoberg AP, Manderson GA, Morgelin M, Day AJ, Heinegard D, Blom AM. Short leucine-rich glycoproteins of the extracellular matrix display diverse patterns of complement interaction and activation. Mol Immunol 2008.
39. Xiang Y, Matsui T, Matsuo K, et al. Comprehensive investigation of disease-specific short peptides in sera from patients with systemic sclerosis: complement C3f-des-arginine, detected predominantly in systemic sclerosis sera, enhances proliferation of vascular endothelial cells. Arthritis Rheum 2007;56:2018-30.
40. Johansen JS, Hvolris J, Hansen M, Backer V, Lorenzen I, Price PA. Serum YKL-40 levels in healthy children and adults. Comparison with serum and synovial fluid levels of YKL-40 in patients with osteoarthritis or trauma of the knee joint. Br J Rheumatol 1996;35:553-9.
41. Manicourt DH, Fujimoto N, Obata K, Thonar EJ. Serum levels of collagenase, stromelysin-1, and TIMP-1. Age- and sex-related differences in normal subjects and relationship to the extent of joint involvement and serum levels of antigenic keratan sulfate in patients with osteoarthritis. Arthritis Rheum 1994;37:1774-83.
42. Mora S, Pitukcheewanont P, Kaufman FR, Nelson JC, Gilsanz V. Biochemical markers of bone turnover and the volume and the density of bone in children at different stages of sexual development. J Bone Miner Res 1999;14:1664-71.

**Table 1: Demographic and clinical characteristics of the patients and controls used in the study ***

| **a) Analysis set (n=150)** | | | | | | | |
|---|---|---|---|---|---|---|---|
| | | | | OA | | | |
| Description | NC | K&L0 | K&L1 | K&L2 | K&L3 | K&L4 | |
| Number of patients | 20 | 44 | 17 | 20 | 23 | 26 | |
| Female, % | 60 | 75 | 71 | 70 | 65 | 35 | |
| Age at entry | 57.5 (47-63) | 57 (34-79) | 59 (51-71) | 60.5 (50-81) | 60 (30-83) | 62.5 (51-78) | |
| Age at onset | / | 44 (25-74) | 50 (16-67) | 47 (23-66) | 51 (17-68) | 41.5 (13-75) | |
| disease duration | / | 6 (1-44) | 8 (1-44) | 11 (1-49) | 10 (1-42) | 20 (3-64) | |
| Body mass index, kg/m² | 25 (19-31) | 25 (19-40) | 26 (18.5-39)23.5 | 23.5 (19-34) | 27 (21-37) | 28 (22.5-45) | |
| Number of joints | | | | | | | |
| early morning stiffness | / | 3 (0-180) | 30 (0-120) | 5 (0-120) | 5 (0-360) | 10(0-120) | |
| % with elevated CRP (> 6 mg/l) | 5 | 10 | 8 | 16 | 18 | 5 | |

| **b) Validation 1 set (n=195)** | | | | | | | |
|---|---|---|---|---|---|---|---|
| | | | | OA | | | |
| Description | NC | K&L0 | K&L1 | K&L2 | K&L3 | K&L4 | RA |
| Number of patients | 16 | 24 | 22 | 32 | 61 | 15 | 25 |
| Female, % | 50 | 58 | 77 | 75 | 48 | 46 | 64 |
| Age at entry | 56 (49-64) | 61 (43 - 79) | 60 (38-78) | 64 (45-92) | 68 (43-81) | 68 (52-76) | 55 (25-77) |
| WOMAC Pain | / | 6(2-20) | 7 (0-20) | 8 (2-15) | 7 (1-14) | 11 (5-17) | / |
| WOMAC Stiffness | / | 4 (0-8) | 4(1-6) | 4(1-7) | 4 (0-6) | 4 (2-7) | / |
| WOMAC Function | / | 21 (2-68) | 27 (0-45) | 27 (6-53) | 24 (1-49) | 34 (18-44) | / |
| Body mass index, kg/m² | 23 (20-30) | 28 (21 - 44) | 31 (23 - 48) | 29 (19 - 42) | 28 (22 - 41) | (23 - 39) | / |
| % with elevated CRP (> 6 mg/l) | / | 21 | 18 | 22 | 10 | 14 | 78 |

| **c) Paired Serum and Synovial fluids (n=19)** | | | | | | | |
|---|---|---|---|---|---|---|---|
| | | | | OA | | | |
| Description | NC | K&L0 | K&L1 | K&L2 | K&L3 | K&L4 | RA |
| Number of patients | / | / | 2 | 3 | 3 | 4 | 7 |
| Female, % | / | / | 0 | 67 | 100 | 100 | 57 |
| Age at entry | / | / | 70 (64-77) | 67 (67-68) | 69 (65-83) | 77 (62-81) | 58 (44-68) |
| Age at onset | / | / | 65 (62-68) | 67 (62-67) | 68 (60-79) | 75 (61-77) | 44 (24-58) |
| disease duration | / | / | 5 (1-10) | 1 (0.5-1) | 3.5 (1-5) | 3 (1-24) | 10 (1.5-20) |
| Body mass index, kg/m² | / | / | 28 (24-33) | 29 (24-35) | 32 (28-34) | 25(25-36) | / |
| % with elevated CRP (> 6 mg/l) | / | / | 0 | 0 | 0 | 0 | 71 |

| | | | | | | | |
|---|---|---|---|---|---|---|---|
| * Except where indicated otherwise, values are the median (range). NC = Negative controls; K&L = Kellgren and Lawrence score; CRP = C-reactive protein; RA = rheumatoid arthritis and WOMAC = Western Ontario and McMaster Universities Osteoarthritis Index. | | | | | | | |

**Table 2: Two statistical analyses, a machine-learning algorithm called Extra-Trees N (Imp %) and a nonparametric Mann-Whitney U test (P-Values), were used for ranking m/z values according to their relative contribution for differentiating K&L0 vs. K&L4 groups in the Analysis, Validation 1 and Validation 2 SELDI-TOF-MS studies. m/z values corresponding to high percentage of information [Imp(%)] and small P values highlight significant different protein concentration between K&L0 vs. K&L4 groups. Ranking of m/z values by both approaches is mentioned in the "Rank" section.**

| **CM10 m/z** | | **Analysis** | | | | **Validation 1** | | | **Validation 2** | | **ID** |
|---|---|---|---|---|---|---|---|---|---|---|---|
| | **mp(%)** | **Rank** | ***P* Value** | **Rank** | **Imp(%)** | **Rank** | ***P* value** | **Rank** | ***P* value** | **Rank** | |
| **3762** | 5.68 | 1 | 1.9 x 10⁻⁷ | 1 | 13.62 | 1 | 1.1 x 10⁻⁶ | 3 | 4.4 x 10⁻³ | 27 | *NI* |
| **2021** | 4.48 | 2 | 2.8 x 10⁻⁵ | 9 | 1.30 | 19 | 2.4 x 10⁻⁴ | 17 | 1.5 x 10⁻⁴ | 14 | Complement C3f |
| **3364** | 3.98 | 3 | 1.4 x 10⁻⁶ | 2 | *NS* | *NS* | *NS* | *NS* | *NS* | *NS* | *NI* |
| **4530** | 3.96 | 4 | 2.1 x 10⁻⁵ | 7 | *NS* | *NS* | *NS* | *NS* | *NS* | *NS* | CTAPIII variant^{†}(2H⁺) |
| **8862** | 3.80 | 5 | 2.0 x 10⁻⁶ | 4 | *NS* | *NS* | *NS* | *NS* | *NS* | *NS* | Beta-TG |
| **9292** | 3.46 | 6 | 3.3 x 10⁻⁵ | 10 | *NS* | *NS* | 3.4 x 10⁻⁴ | 27 | 0.01 | *NS* | CTAPIII |
| **9060** | 2.70 | 7 | 2.3 x 10⁻⁵ | 8 | *NS* | *NS* | *NS* | *NS* | *NS* | *NS* | CTAPIII variant |
| **11238** | 2.68 | 8 | 1.8 x 10⁻³ | 22 | *NS* | *NS* | *NS* | *NS* | *NS* | *NS* | *NI* |
| **7766** | 2.59 | 9 | 3.7 x 10⁻⁵ | 11 | *NS* | *NS* | *NS* | *NS* | *NS* | *NS* | PF4 |
| **4430** | 2.18 | 11 | 5.6 x 10⁻⁶ | 5 | *NS* | *NS* | *NS* | *NS* | *NS* | *NS* | Beta-TG^{†}(2H⁺) |
| **3157** | 1.99 | 12 | 7.5 x 10⁻⁵ | 13 | *NS* | *NS* | *NS* | *NS* | *NS* | *NS* | *NI* |
| **4644** | 1.98 | 13 | 4.7 x 10⁻⁵ | 12 | *NS* | *NS* | 3.8 x 10⁻² | 64 | *NS* | *NS* | CTAPIII^{†}(2H⁺) |
| **1979** | 1.21 | 19 | 1.7 x 10⁻³ | 21 | 2.27 | 8 | 3.8 x 10⁻⁶ | 6 | 1.0 x 10⁻³ | 23 | V65 Vitronectin fragment |
| | | | | | | | | | | | |

| **IMAC m/z** | | **Analysis** | | | | | | | **Validation 2** | | **ID** |
|---|---|---|---|---|---|---|---|---|---|---|---|
| | **Imp(%)** | **Rank** | ***P* value** | **Rank** | | | | | ***P* value** | **Rank** | |
| **4286** | 5.64 | 1 | 3.9 x 10⁻⁴ | 3 | | | | | NS | NS | *NI* |
| **11683** | 4.45 | 2 | 8.8 x 10⁻⁵ | 1 | | | | | NS | NS | SAA |
| **2021** | 4.22 | 3 | 3.2 x 10⁻⁴ | 4 | | | | | 2.0 x 10⁻⁶ | 11 | Complement C3f |
| **1691** | *ND* | *ND* | *ND* | *ND* | | | | | 1.6 x 10⁻⁷ | 6 | Complement C3f |
| **1778** | *ND* | *ND* | *ND* | *ND* | | | | | 2.2 x 10⁻⁷ | 8 | Complement C3f |
| **1865** | *ND* | *ND* | *ND* | *ND* | | | | | 1.2 x 10⁻⁶ | 9 | Complement C3f |

| | | | | | | | | | | | |
|---|---|---|---|---|---|---|---|---|---|---|---|
| *NI*: Not Identified *ND*: Not Detected due to the use of SPA matrix allowing peak detection only from 2000 m/z value *NS*: Not statistically Significant †: Doubly charged form (2H+) | | | | | | | | | | | |

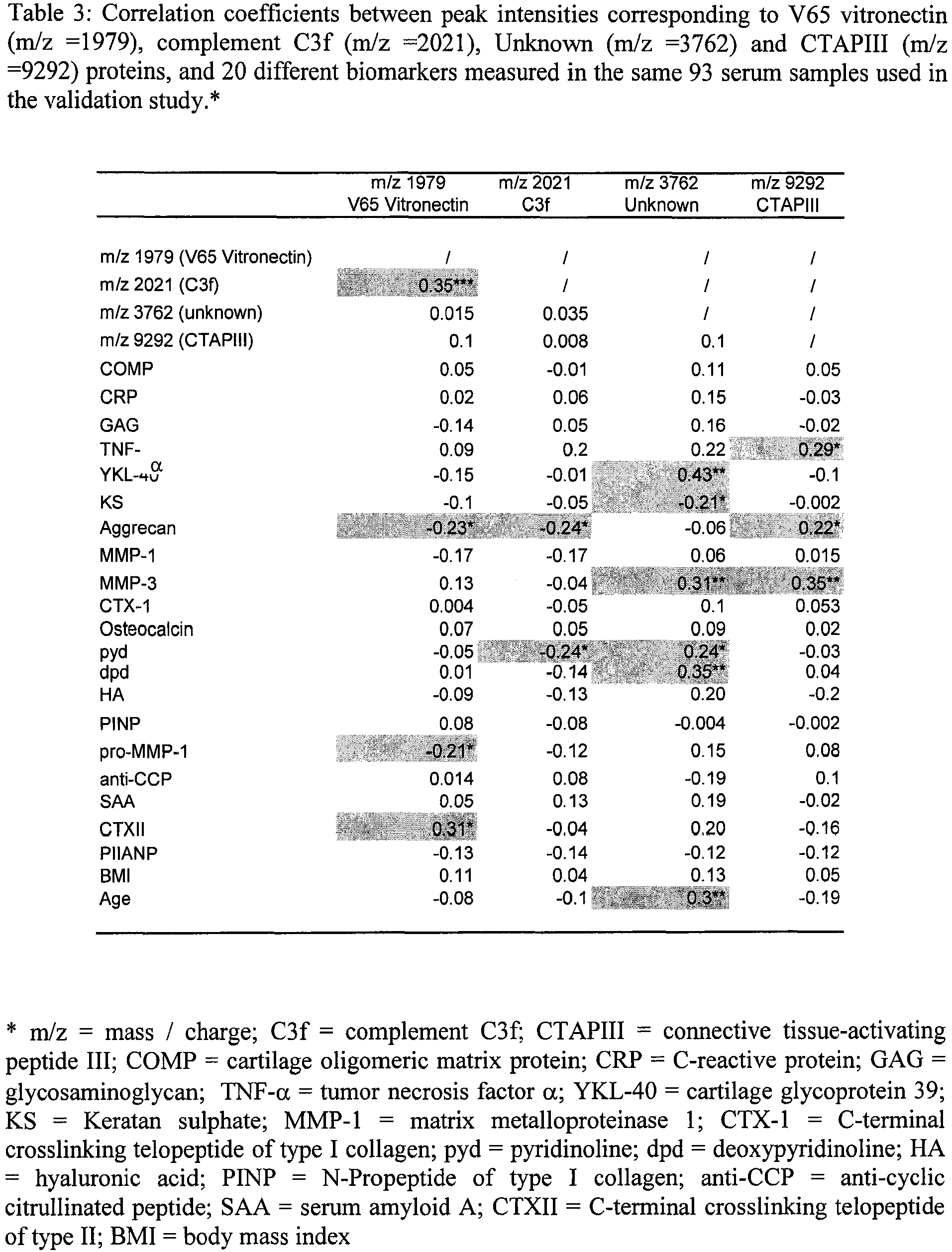

## Claims

1. A method of diagnosing osteoarthritis or monitoring the disease progression of osteoarthritis, comprising identifying the presence of an increased concentration of a marker selected from V65 vitronectin fragment or fragments, variants or degradation products thereof in a sample obtained from a subject, wherein the V65 vitronectin fragment or fragments, variants or degradation products thereof comprise a C-terminal end product of the V65 vitronectin subunit in the heparin binding domain.

2. A method according to claim 1, wherein the sample is blood, serum, urine or synovial fluid.

3. A method according to any preceding claim, comprising identifying the presence of an increased concentration of the C-terminal V65 vitronectin fragment or a fragment, variant or degradation product thereof in the sample.

4. A method according to any of claims 1 to 3, wherein the C-terminal V65 vitronectin fragment comprises the following amino acid sequence: SQRGHSRGRNQNSRRPS.

5. A method according to any preceding claim, further comprising identifying the presence of an increased concentration of complement fragment C3f or a fragment, variant or degradation product thereof in the sample.

6. A method according to any claim 5, wherein the complement fragment C3f fragment or variant comprises one of the following amino acid sequences: SSKITHRIHWESASLLR, SSKITHRIHWESASLL, SSKITHRIHWESASL, SSKITHRIHWESAS and SSKITHRIHWESA

7. A method according to any preceding claim, further comprising identifying the presence of a decreased concentration of CTAPIII protein or a fragment, variant or degradation product thereof in the sample.

8. A method according to any claim 7, wherein the CTAPIII protein comprises one of the following amino acid sequences:

NLAKGKEESLDSDLYAELRCMCIKTTSGIHPKNIQSLEVIGKGTHCNQVEVIAT
LKD GRKICLDPDA PRIKKIVQKK LAGDESAD,
LAKGKEESLDSDLYAELRCMCIKTTSGIHPKNIQSLEVIGKGTHCNQVEVIATL
KD GRKICLDPDA PRIKKIVQKK LAGDESAD,
AKGKEESLDSDLYAELRCMCIKTTSGIHPKNIQSLEVIGKGTHCNQVEVIATLK
D GRKICLDPDA PRIKKIVQKK LAGDESAD,
KGKEESLDSDLYAELRCMCIKTTSGIHPKNIQSLEVIGKGTHCNQVEVIATLKD
GRKICLDPDA PRIKKIVQKK LAGDESAD and
GKEESLDSDLYAELRCMCIKTTSGIHPKNIQSLEVIGKGTHCNQVEVIATLKD
GRKICLDPDA PRIKKIVQKK LAGDESAD.

9. A method according to any of claims 5 to 8, comprising identifying at least two markers in the sample.

10. A method according to claim 9, comprising identifying at least the presence of an increased concentration of V65 vitronectin fragment or fragments, variants or degradation products thereof and the presence of an increased concentration of complement fragment C3f or fragments, variants or degradation products thereof in the sample.

11. A method according to claim 10, also comprising identifying the presence of a decreased concentration of CTAPIII protein or a fragment, variant or degradation product thereof in the sample.

12. A method according to any preceding claim further comprising identifying the presence or absence, increased concentration or decreased concentration of any other marker of osteoarthritis.

## Patentansprüche

1. Verfahren zur Diagnose von Osteoarthritis oder zum Überwachen des Krankheitsfortschritts von Osteoarthritis, mit dem Schritt zum Identifizieren des Vorhandenseins einer erhöhten Konzentration eines Markers, der ausgewählt ist aus einem V65-Vitronektin-Fragment oder von Fragmenten, Varianten oder Abbauprodukten davon in einer von einer Person erhaltenen Probe, wobei das V65-Vitronektin-Fragment oder die Fragmente, Varianten oder Abbauprodukte davon ein Produkt mit einem C-terminalen Ende der V65-Vitronektin-Untereinheit in der Heparin-bindenden Domäne aufweisen.

2. Verfahren nach Anspruch 1, wobei die Probe Blut, Serum, Urin oder Synovialflüssigkeit ist.

3. Verfahren nach Anspruch 1 oder 2, mit dem Identifizieren des Vorhandenseins einer erhöhten Konzentration des C-terminalen V65-Vitronektin-Fragments oder eines Fragments, einer Variante oder eines Abbauprodukts davon in der Probe.

4. Verfahren nach einem der Ansprüche 1 bis 3, wobei das C-terminale V65-Vitronektin-Fragment die folgende Aminosäuresequenz aufweist: SQRGHSRGRNQNSRRPS.

5. Verfahren nach einem der vorangehenden Ansprüche, ferner mit dem Identifizieren des Vorhandenseins einer erhöhten Konzentration eines Komplementfragments C3f oder eines Fragments, einer Variante oder eines Abbauprodukts davon in der Probe.

6. Verfahren nach Anspruch 5, wobei das Komplementfragment C3f, ein Fragment oder eine Variante davon eine der folgenden Aminosäuresequenzen aufweist: SSKITHRIHWESASLLR, SSKITHRIHWESASLL,
SSKITHRIHWESASL, SSKITHRIHWESAS und SSKITHRIHWESA.

7. Verfahren nach einem der vorangehenden Ansprüche, ferner mit dem Identifizieren des Vorhandenseins einer verminderten Konzentration eines CTAPIII-Proteins oder eines Fragments, einer Variante oder eines Abbauprodukts davon in der Probe.

8. Verfahren nach Anspruch 7, wobei das CTAPIII-Protein eine der folgenden Aminosäuresequenzen aufweist:

NLAKGKEESLDSDLYAELRCMCIKTTSGIHPKNIQSLEVIGKGTHCNQVEVIAT
LKD GRKICLDPDA PRIKKIVQKK LAGDESAD,
LAKGKEESLDSDLYAELRCMCIKTTSGIHPKNIQSLEVIGKGTHCNQVEVIATL
KD GRKICLDPDA PRIKKIVQKK LAGDESAD,
AKGKEESLDSDLYAELRCMCIKTTSGIHPKNIQSLEVIGKGTHCNQVEVIATLK
D GRKICLDPDA PRIKKIVQKK LAGDESAD,
KGKEESLDSDLYAELRCMCIKTTSGIHPKNIQSLEVIGKGTHCNQVEVIATLKD
GRKICLDPDA PRIKKIVQKK LAGDESAD und
GKEESLDSDLYAELRCMCIKTTSGIHPKNIQSLEVIGKGTHCNQVEVIATLKD
GRKICLDPDA PRIKKIVQKK LAGDESAD.

9. Verfahren nach einem der Ansprüche 5 bis 8, mit dem Identifizieren mindestens zweier Marker in der Probe.

10. Verfahren nach Anspruch 9, mit dem Identifizieren des Vorhandenseins einer erhöhten Konzentration eines V65-Vitronektin-Fragments oder von Fragmenten, Varianten oder Abbauprodukten davon und/oder des Vorhandenseins einer erhöhten Konzentration eines Komplementfragments C3f oder von Fragmenten, Varianten oder Abbauprodukten davon in der Probe.

11. Verfahren nach Anspruch 10, ferner mit den Identifizieren des Vorhandenseins einer verminderten Konzentration eines CTAPIII-Proteins oder eines Fragments, einer Variante oder eines Abbauprodukts davon in der Probe.

12. Verfahren nach einem der vorangehenden Ansprüche, ferner mit dem Identifizieren des Vorhandenseins oder Nichtvorhandenseins einer erhöhten Konzentration oder einer verminderten Konzentration irgendeines anderen Markers von Osteoarthritis.

## Revendications

1. Méthode pour diagnostiquer l'arthrose ou surveiller la progression morbide de l'arthrose, comprenant l'identification de la présence d'une concentration accrue d'un marqueur choisi parmi un fragment de vitronectine V65, et des fragments, variants et produits de dégradation de celui-ci, dans un échantillon obtenu à partir d'un sujet, dans laquelle le fragment de vitronectine V65 ou les fragments, variants ou produits de dégradation de celui-ci comprennent un produit d'extrémité C-terminale de la sous-unité de vitronectine V65 dans le domaine se liant à l'héparine.

2. Méthode selon la revendication 1, dans laquelle l'échantillon est du sang, du sérum, de l'urine ou du liquide synovial.

3. Méthode selon l'une quelconque des revendications précédentes, identifiant la présence d'une concentration accrue du fragment de vitronectine V-65 C-terminal ou d'un fragment, variant ou produit de dégradation de celui-ci dans l'échantillon.

4. Méthode selon l'une quelconque des revendications 1 à 3, dans laquelle le fragment de vitronectine V65 C-terminal comprend la séquence d'acides aminés suivante : SQRGHSRGRNQNSRRPS.

5. Méthode selon l'une quelconque des revendications précédentes, comprenant en outre l'identification de la présence d'une concentration accrue du fragment de complément C3f ou d'un fragment, variant ou produit de dégradation de celui-ci dans l'échantillon.

6. Méthode selon la revendication 5, dans laquelle le fragment de complément C3f ou le fragment ou variant de celui-ci comprend l'une des séquences d'acides aminés suivantes : SSKITHRIHWESASLLR, SSKITHRIHWESASLL, SSKITHRIHWESASL, SSKITHRIHWESAS et SSKITHRIHWESA.

7. Méthode selon l'une quelconque des revendications précédentes, comprenant en outre l'identification de la présence d'une concentration réduite de protéine CTAPIII ou d'un fragment, variant ou produit de dégradation de celle-ci dans l'échantillon.

8. Méthode selon la revendication 7, dans laquelle la protéine CTAPIII comprend une ou plusieurs des séquences d'acides aminés suivantes :

NLAKGKEESLDSDLYAELRCMCIKTTSGIHPKNIQSLEVIGKGTHCNQVEVIATLKD
GRKICLDPDA PRIKKIVQKK LAGDESAD,
LAKGKEESLDSDLYAELRCMCIKTTSGIHPKNIQSLEVIGKGTHCNQVEVIATLKD
GRKICLDPDA PRIKKIVQKK LAGDESAD,
AKGKEESLDSDLYAELRCMCIKTTSGIHPKNIQSLEVIGKGTHCNQVEVIATLKD
GRKICLDPDA PRIKKIVQKK LAGDESAD,
KGKEESLDSDLYAELRCMCIKTTSGIHPKNIQSLEVIGKGTHCNQVEVIATLKD
GRKICLDPDA PRIKKIVQKK LAGDESAD et
GKEESLDSDLYAELRCMCIKTTSGIHPKNIQSLEVIGKGTHCNQVEVIATLKD
GRKICLDPDA PRIKKIVQKK LAGDESAD.

9. Méthode selon l'une quelconque des revendications 5 à 8, comprenant l'identification d'au moins deux marqueurs dans l'échantillon.

10. Méthode selon la revendication 9, comprenant l'identification au moins de la présence d'une concentration accrue de fragment de vitronectine V65 ou de fragments, variants ou produits de dégradation de celui-ci et de la présence d'une concentration accrue de fragment de complément C3f ou de fragments, variants ou produits de dégradation de celui-ci dans l'échantillon.

11. Méthode selon la revendication 10, comprenant aussi l'identification de la présence d'une concentration réduite de protéine CTAPIII ou d'un fragment, variant ou produit de dégradation de celle-ci dans l'échantillon.

12. Méthode selon l'une quelconque des revendications précédentes, comprenant en outre l'identification de la présence ou de l'absence d'une concentration accrue ou d'une concentration réduite de n'importe quel autre marqueur de l'arthrose.
